# EUROPEAN PATENT SPECIFICATION

(11) **EP 2 144 058 B1**
(45) Date of publication and mention of the grant of the patent: **10.06.2015**
(21) Application number: 08748426.7
(22) Date of filing: 28.04.2008
(51) Int. Cl.: G01N 30/36, G01N 30/72, G01N 33/49, G01N 33/15, G01N 33/94

(54) **DETECTION OF BLOOD PLASMA AMYGDALIN OF DISSIPATING BLOOD STASIS BOTANICAL**
NACHWEIS VON PLASMAAMYGDALIN EINER BLUTSTASEAUFLÖSENDEN PFLANZE
DÉTECTION D'AMYGDALINE DE PLASMA SANGUIN D'UN EXTRAIT DE PLANTE ÉLIMINANT L'HÉMOSTASE

(30) Priority: 27.04.2007 CN 200710040139
(43) Date of publication of application: 13.01.2010
(73) Proprietor: Shanghai Sundise Chinese Medicine Technology Development Co., Ltd, Shanghai 200051 (CN); Ma, Yueming, Shanghai 200051 (CN); Wang, Tianming, Shanghai 200051 (CN); Zhang, Yongyu, Shanghai 200051 (CN)
(72) Inventor: MA, Yueming, Shanghai 200051 (CN); WANG, Tianming, Shanghai 200051 (CN); ZHANG, Yongyu, Shanghai 200051 (CN)
(74) Representative: Pfenning, Meinig & Partner GbR
(86) International application number: PCT/CN2008/000867
(87) International publication number: WO 2008/131649

(56) References cited:
- WO-A1-2004/014409
- WO-A1-2004/014409
- KR-A- 20060 038 027
- XINFENG ZHAO ET AL: "HPLC with Column Switching Coupled to APCI-MS for Pharmacokinetic Study of Amygdalin in Rabbit Plasma" CHROMATOGRAPHIA ; AN INTERNATIONAL JOURNAL FOR RAPID COMMUNICATION IN CHROMATOGRAPHY, ELECTROPHORESIS AND ASSOCIATED TECHNIQUES, VIEWEG VERLAG, WI, vol. 65, no. 3-4, 15 December 2006 (2006-12-15), pages 149-153, XP019476696 ISSN: 1612-1112
- KANG S H ET AL: "Micellar electrokinetic chromatography for the analysis of d-amygdalin and its epimer in apricot kernel" JOURNAL OF CHROMATOGRAPHY, ELSEVIER SCIENCE PUBLISHERS B.V, NL LNKD- DOI:10.1016/S0021-9673(99)01107-3, vol. 866, no. 2, 14 January 2000 (2000-01-14), pages 253-259, XP004244468 ISSN: 0021-9673
- CHURCHWELL ET AL: "Improving LC-MS sensitivity through increases in chromatographic performance: Comparisons of UPLC-ES/MS/MS to HPLC-ES/MS/MS" JOURNAL OF CHROMATOGRAPHY B: BIOMEDICAL SCIENCES & APPLICATIONS, ELSEVIER, AMSTERDAM, NL LNKD- DOI:10.1016/J.JCHROMB.2005.05.037, vol. 825, no. 2, 25 October 2005 (2005-10-25), pages 134-143, XP005102436 ISSN: 1570-0232
- LIAO Z. ET AL: 'The study in situ on rat intestinal absorption of the active components in GuizhiFuling capsule' CHIN. J. NAT. MED. vol. 3, no. 5, September 2005, pages 303 - 307, XP008126204
- XIE H. ET AL: 'Determination of anthraquinones and amygdalin in' SH. J. TCM vol. 40, no. 7, July 2006, pages 73 - 76, XP008126206
- WATERS: 'Oasis HLB cartridge 6cc/150mg 30mym 30/box [186003365]', [Online] 2010, Retrieved from the Internet: <URL:http://www.waters.com/waters> [retrieved on 2010-00-00]
- WATERS: 'ACQUITY UPLC BEH Columns', [Online] Retrieved from the Internet: <URL:http://www.waters.com/waters> [retrieved on 2010-00-00]

## Description

### Technical Field

This invention belongs to the field of pharmacokinetics, particularly involves the determination method of blood plasma Amygdalin.

### Background Art

Botanical compositions strengthening body resistance and dissipating blood stasis are composed of compound prescriptions including salvia miltiorrhiza, peach kernel, Schisandra chinens etc., which have the effect of curing liver, lung and kidney fibrosis; however, due to lack of pharmacokinetics research has been carried out on botanical compositions strengthening body resistance and dissipating blood stasis botanical, so it is not clear about the effective ingredients in vivo, and it is difficult to provide a basis for quality control and guiding clinical rational administration, thus hinder those drugs from entering the international market.

So far, no relevant pharmacokinetic research report on botanical compositions strengthening body resistance and dissipating blood stasis has been found, and there are not many detection method of Amygdalin of the compound prescription in biological samples (including blood plasma sample).

Zhao et al. (X. Zhao, W. Zang, X. Zhao, S. Wang, X. Zheng, J. Zheng, "HPLC with Column Switching Coupled to APCI-MS for Pharmacokinetic Study of Amygdalin in Rabbit Plasma", Chromatographia, 2007, 65, 149-153) describes a fast validated assay for the pharmacokinetic study or amygdalinin *Armeniacae Semen* in rabbit. The method involved column switching (CS) enrichment, separation, post-column derivatization, and atmospheric pressure chemical ionization (APCI) mass spectrometric detection.

WO 2004/014409 A1 relates to the developing method for separating D-amygdalin and ncoamygdalin by reversed-phase HPLC method and optimum condition for inhibition of racemization of amygdalin.

Kang et al. (S. H. Kang, H. Jung, N. Kim, D.-H. Shin, D. S. Chung, "Micellar electrokinetic chromatography for the analysis of D-amygdalin and its epimer in apricot kernel", Journal of Chromatography A, 2000, 866, 253-259) describes a simple, rapid and reproducible method for the determination of D-amygdalin and its epimer by using micellar electrokinetic chromatography (MEKC).

Churchwell et al. (M. I. Churchwell, N. C. Twaddle, L. R. Meeker, D. R. Doerge, "Improving LC-MS sensitivity through increases in chromatographic performance: Comparisons of UPLC-ES/MS/MS to HPLC-ES/MS/MS", Journal of Chromatography B, 2005, 825, 134-143) explores the differences in LC-MS performance by conducting a side-by-side comparison of UPLC for several methods previously optimized for HPLC-based separation and quantification of multiple analytes with maximum throughput.

### Contents of the Invention

The technical matters aim to be resolved by this invention is to provide a detection method of blood plasma Amygdalin of strengthening body resistance and dissipating blood stasis botanical, and the method is used for pharmacokinetics research, and clarifying the pharmacokinetics rules of the blood plasma Amygdalin.

The technical matters solved by this invention arc achieved through the following technical solutions:

The detection method of blood plasma Amygdalin includes the following steps:
(1) Pretreatment of mammalian blood plasma samples
   a. Collect mammal plasma containing drugs after being administered the strengthening body resistance and dissipating blood stasis botanical, misce bene after adding 2- 5 mol / L phosphoric acid, and the volume ratio between the blood plasma and phosphoric acid is 1:2-3, applying the blood plasma with drugs to small column of Waters Oasis HLB activated by methanol and water; after leaching with water and 80-100% methanol and eluting with 0.2-1% ammonia -methanol, drying and enriching the eluent under the condition of 25-30 °C, and redissolving the eluent with mobile phase.
   b. Detection with UPLC-MS method;
(2). UPLC/MS detection method

UPLC condition: chromatographic column: Acquity UPLC BEH C18, 2.1 x 100mm, mobile phase A: water- Acetonitrile- Formic acid 95:5:0.1 v/v/v, mobile phase B: Acetomtrile-Formic acid 100: The electrospray ionization (ESI) ion source of the described step (2), detects with positive ion mode, the desolvation gas flow is 440 L/h, the desolvation gas temperature is 300 °C, the cone gas flow is 50 L/h, the ion source temperature is 100 °C, the spray capillary voltage is 3800 V, the sampling cone voltage is 30V, the extracting cone voltage is 2.00 V, the lens voltage is 0.1 V, and mass scanning at the range of m/z 150-800.

In solid-phase extraction process of this invention, the adsorption capacity of the solid phase to the analytes is greater than the sample mother liquor, when the samples pass through the solid-phase extraction column, the analytes and a number of similar ingredients were adsorbed on the surface of solid, and other ingredients then pass through the column with the sample mother liquor, first leach columns with larger polar solvents, to rinse and remove a number of unrelated ingredients, and finally elute the analytes with appropriate solvent, drain and enrich the eluent; use UPLC system, separate the analytes with other ingredients in the elution, and finally detected with mass spectrometry detector.

The Amygdalin in this invention is a water-soluble ingredient, using solid-phase extraction method, can fully extract the Amygdalin in the samples, combined with using a UPLC/MS system to detect, markedly improve the resolutions of Amygdalin among other ingredients in the samples, and the analysis method is more sensitive and faster, to facilitate the detection of the blood plasma concentration of the Amygdalin in pharmacokinetic research.

### Description of Figures

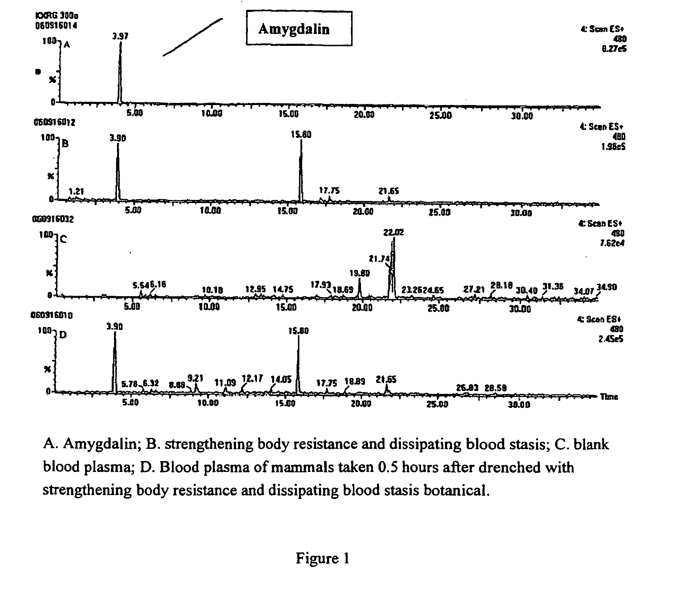

Figure 1. UPLC-MS chromatogram of Amygdalin.

### Mode of Carrying out the Invention

Combining with the specific embodiments, further elaboration of this invention is given below. It should be understood that these embodiments are only for description of the present invention but not for the use of limiting the scope of the present invention. It should also be understood, after reading the contents taught in this invention, technicians in this field can make various changes or modification to this invention.

### Embodiment 1

A detection method of blood plasma Amygdalin.
1. Method of pretreating mammalian blood plasma sample: Collect mammal plasma containing drugs after being administered the strengthening body resistance and dissipating blood stasis botanical, misce bene after adding 2- 5 mol/L phosphoric acid, and the volume ratio between the biological sample and phosphoric acid is 1:2-3, applying the blood plasma with drugs to small column of Waters Oasis HLB activated by methanol and water; after leaching with water and 80-100% methanol and eluting with 0.2-1% ammonia -methanol, drying and enriching the eluent under the condition of 25 to 30 °C, and redissolving the eluent with mobile phase.
2. UPLC / MS determination method: The analysis conditions of the applied UPLC/ MS method in this invention, UPLC condition: chromatographic column: Acquity UPLC BEH C18, 2.1 × 100 mm, mobile phase A: Water-Acetonitrile-Formic acid 95:5: 0.1 v/v/v, mobile phase B: Acetomtrile- Formic acid 100:0.1 v/v, eluting in accordance with the following gradient:

| | Time (min) | Flow Rate (mL/min) | % A | % B |
|---|---|---|---|---|
| | 0 | 0.300 | 100 | 0 |
| | 5.00 | 0.300 | 85.0 | 15.0 |
| | 10.00 | 0.300 | 70.0 | 30.0 |
| MS | 20.00 | 0.300 | 40.0 | 60.0 |
| | 30.00 | 0.300 | 20.0 | 80.0 |
| | 35.00 | 0.300 | 20.0 | 80.0 |
| | 35.01 | 0.300 | 100.0 | 0.0 |
| | 38.00 | 0.300 | 100.0 | 0.0 |

conditions: electrospray ionization(ESI) ion source, detecting with positive ion mode, the desolvation gas flow is 440 L/h, the desolvation gas temperature is 300 °C, the cone gas flow is 50 L/h, the ion source temperature is 100 °C, the spray capillary voltage is 3800 V, the sampling cone voltage is 30V, the extracting cone voltage is 2.00 V, the lens voltage is 0.1 V, and mass scanning at the range of m/z 150 to 800.
Detection results: Amygdalin can be detected in the blood plasma of mammals after application of botanical compositions strengthening body resistance and dissipating blood stasis (see Figure 1).

## Claims

1. In vitro method for detecting blood plasma Amygdalin including the following steps:
(1) pretreatment of mammalian blood plasma samples including
a. In Collected mammal blood plasma containing drugs of botanical compositions strengthening body resistance and dissipating blood stasis after being administered, mix well after adding 2 to 5 mol/L phosphoric acid, the volume ratio between the blood plasma and phosphoric acid being 1:2-3, applying the blood plasma to a column of Waters Oasis HLB activated by methanol and water; leaching with water and 80-100% methanol; eluting with 0.2-1% ammonia -methanol, drying and enriching the eluent under the condition of 25 to 30 °C, and redissolving the eluent with a mobile phase;
(2) detection of amygdalin with UPLC/MS method using the following measurement conditions:
UPLC condition: chromatographic column: Acquity UPLC BEH C18, 2.1 x 100 mm, mobile phase A: water- Acetonitrile- Formic acid 95:5:0.1 v/v/v, mobile phase B: Acetonitrile-Formic acid 100: 0.1 v/v;
MS condition: electrospray ionization (ESI) ion source, detecting with positive ion mode, and mass scanning at the range of m/z 150-800,
wherein the step of detecting amygdalin with UPLC/MS method detects with positive ion mode, the desolvation gas flow is 440 L/h, the desolvation gas temperature is 300 °C, the cone gas flow is 50 L/h, the ion source temperature is 100 °C, the spray capillary voltage is 3800 V, the sampling cone voltage is 30V, the extracting cone voltage is 2.00 V, the lens voltage is 0.1 V, and mass scanning at the range of m/z 150 to 800.

## Patentansprüche

1. In-vitro-Verfahren zur Detektion von Blutplasma-Amygdalin, umfassend die folgenden Schritte:
(1) Vorbehandlung von Säugetierblutplasma-Proben, enthaltend
a. In gesammeltem Säugetierblutplasma enthaltend Medikamente pflanzlicher Zusammensetzungen, welche nach Verabreichung die Abwehrkraft des Körpers stärken und Blutstase dissipieren, gutes Mischen nach Hinzugeben von 2 bis 5 mol/L Phosphorsäure, wobei das Volumenverhältnis zwischen dem Blutplasma und der Phosphorsäure 1:2-3 beträgt, Aufgabe des Blutplasmas auf eine durch Methanol und Wasser aktivierte Säule Waters Oasis HLB; Waschen mit Wasser und 80-100 % Methanol; Eluieren mit 0,2-1 % Ammoniak-Methanol, Trocknen und Anreichern des Eluenten bei 25 bis 30 °C, und Wiederauflösen des Eluenten mit einer mobilen Phase;
(2) Detektion von Amygdalin mit einem UPLC/MS Verfahren, wobei die folgenden Messparameter angewandt werden:
UPLC-Parameter: chromatographische Säule: Acquity UPLC BEH C18, 2,1 x 100 mm, mobile Phase A: Wasser-Acetonitril-Ameisensäure 95:5:0,1 v/v/v, mobile Phase B: Acetonitril-Ameisensäure 100:0,1 v/v;
MS-Parameter: Elektrospray-Ionisations (ESI) Ionen-Quelle, Detektion im positiven Ionenmodus, und Massen-Scan im Bereich von m/z 150-800,
wobei der Schritt der Detektion von Amygdalin mit einem UPLC/MS Verfahren im positiven Ionenmodus detektiert, der Desolvatisierungsgasfluss 440 L/h beträgt, die Desolvatisierungsgastemperatur 300 °C beträgt, der Düsengasstrom 50 L/h beträgt, die Ionenquellentemperatur 100 °C beträgt, die Sprayka-pillarspannung 3800 V beträgt, die Sampling-Cone-Spannung 30 V beträgt, die Extracting-Cone-Spannung 2,00 V beträgt, die Linsenspannung 0,1 V beträgt, und der Massen-Scan im Bereich von m/z 150 bis 800 durchgeführt wird.

## Revendications

1. Procédé *in vitro* pour détecter l'amygdaline plasmatique comprenant les étapes suivantes :
(1) prétraitement d'échantillon de plasma sanguin de mammifère comprenant
a. dans du plasma sanguin collecté contenant des médicaments de compositions botaniques renforçant la résistance du corps et dissipant la stase sanguine après administration, mélanger soigneusement après ajout de 2 à 5 mol/l d'acide phosphorique, le rapport de volume entre le plasma sanguin et l'acide phosphorique étant de 1:2-3, l'application du plasma sanguin sur une colonne Waters Oasis HLB activée par du méthanol et de l'eau ; rinçage avec de l'eau et 80 à 100 % de méthanol ; l'élution avec 0,2 à 1 % d'ammoniac-méthanol, la déshydratation et l'enrichissement de l'éluant dans la condition de 25 à 30 °C, et la redissolution de l'éluant avec une phase mobile ;
(2) détection de l'amygdaline avec un procédé UPLC/MS en utilisant les conditions de mesure suivantes :
conditions UPLC: colonne chromatographique : Acquity UPLC BEH C18, 2,1 x 100 mm, phase mobile A : eau-acétonitrile-acide formique 95:5:0,1 v/v/v, phase mobile B : acétonitrile-acide formique 100:0,1 % v/v ;
conditions MS : source d'ions ionisation electrospray (ESI), détection avec mode d'ions positifs, et balayage de masse dans la plage de m/z de 150 à 800,
dans lequel l'étape de détection de l'amygdaline avec le procédé UPLC/MS détecte avec le mode d'ions positifs, le débit de gaz de désolvatation est de 440 l/h, la température de gaz de désolvatation est de 300 °C, le débit de gaz de cône est de 50 l/h, la température de source d'ions est de 100 °C, la tension de capillaire de pulvérisation est de 3800 V, la tension de cône d'échantillonnage est de 30 V, la tension de cône d'extraction est de 2,00 V, la tension de lentille est de 0,1 V, et le balayage de masse dans la plage de m/z de 150 à 800.
